# EUROPÄISCHE PATENTSCHRIFT

(11) **EP 2 520 235 B1**
(45) Veröffentlichungstag und Bekanntmachung des Hinweises auf die Patenterteilung: **20.04.2016**
(21) Anmeldenummer: 12166441.1
(22) Anmeldetag: 02.05.2012
(51) Int. Cl.: A61B 17/16

(54) **Chirurgisches Handstück**
Surgical hand-held tool
Pièce à main chirurgicale

(30) Priorität: 06.05.2011 DE 102011050191
(43) Veröffentlichungstag der Anmeldung: 07.11.2012
(73) Patentinhaber: AESCULAP AG, 78532 Tuttlingen (DE)
(72) Erfinder: Hoegerle, Roland-Alois, 78532 Tuttlingen (DE); Mattes, Uwe, 78532 Tuttlingen (DE); Blust, Edgar, 78126 Königsfeld (DE); Pfister, Ralf, 78647 Trossingen (DE)
(74) Vertreter: Hoeger, Stellrecht & Partner Patentanwälte mbB

(56) Entgegenhaltungen:
- WO-A1-2009/109198
- US-A1- 2003 000 774
- US-A1- 2005 203 487
- US-A1- 2010 262 145

## Beschreibung

Die vorliegende Erfindung betrifft ein chirurgisches Handstück zum Antreiben eines chirurgischen Werkzeugs, mit einem Gehäuse, in welchem ein Antrieb in Form eines Druckluftmotors und mindestens ein abtriebsseitig mit dem Antrieb zusammenwirkendes Lager- oder Getriebeteil angeordnet sind, welches Handstück mindestens einen Schmiermittelsprayanschluss umfasst zum Koppeln mit einer Schmiermittelsprayquelle und mindestens eine Schmiermittelsprayverbindungsleitung zum Ausbilden einer Fluidverbindung zwischen dem mindestens einen Schmiermittelsprayanschluss und dem mindestens einen Lager-oder Getriebeteil, welcher Schmiermittelsprayanschluss mit dem mindestens einen Lager- oder Getriebeteil in Fluidverbindung steht, wobei der mindestens eine Schmiermittelsprayanschluss proximalseitig des Antriebs angeordnet oder ausgebildet ist.

Chirurgische Motorensysteme sind in modularer sowie auch integrierter Bauweise bekannt. Beim modularen Aufbau ist das Handstück, welches mit einem Bearbeitungswerkzeug zum Bewegen desselben gekoppelt werden kann, vom Antrieb getrennt. Das Handstück umfasst dann allenfalls mindestens ein Lager- oder Getriebeteil. Zur Verbindung eines solchen Handstücks mit dem Antrieb dient eine Kupplung. Mit anderen Worten können das Handstück und der Motor zerlegt werden. Dadurch können Komponenten des Motorensystems besser gereinigt werden, da innere Hohlräume derselben besser zugänglich sind. Allerdings hat ein modularer Aufbau auch Nachteile. Die Kupplung zwischen dem Handstück und dem Antrieb führt zu einer Verlängerung der aus Handstück und Antrieb gebildeten Einheit. Ferner bildet die Kupplung eine Fehlerquelle, und zwar sowohl was die Herstellung als auch die Benutzung der Komponenten anbelangt.

Im Gegensatz hierzu bietet eine integrierte Bauweise eines chirurgischen Handstücks, welches nicht nur das mindestens eine Lager- oder Getriebeteil, sondern auch noch den Antrieb umfasst, Vorteile. Ein derartiges Handstück kann kleiner und kompakter ausgebildet werden. Ferner wird durch die Integration des Antriebs in das Handstück Gewicht eingespart. Antriebs- und Abtriebswelle lassen sich ferner zu einem Antriebsstrang vereinigen. Da Lagerstellen oder ein Spielt im Bereich einer Kupplung zwischen einem nur Lageroder Getriebeteile umfassenden Handstück und Antrieb aufgrund der integrierten Bauweise nicht auftreten können, wird zudem die Laufruhe verbessert.

Damit Lager- und Getriebeteile des Handstücks dauerhaft einsatzfähig sind, kann das Handstück entweder komplett abgedichtet oder in einer sogenannten offenen Bauweise ausgebildet werden. Im Falle einer Abdichtung sind sämtliche Lager- oder Getriebeteile abgedichtet und mit einer Dauerschmierung versehen.

Die offene Bauweise hat jedoch insbesondere den Nachteil, dass nach jeder Reinigung und vor jeder Sterilisation Lager- und Getriebeteile geschmiert werden müssen. Bei einem auf Lebensdauer abgedichteten Gehäuse ist dies nicht erforderlich. Bei einem Antrieb in Form eines Druckluftmotors muss dieser systembedingt abtriebsseitig abgedichtet sein. Eine Schmierung eines Handstücks erfolgt jedoch von der sogenannten "reinen" zur sogenannten "unreinen" Seite hin. Die unreine Seite des Handstücks bildet das mit dem Werkzeug koppelbare Ende. Die reine Seite bildet die Antriebsseite des Motors. Eine Schmierung abtriebsseitiger Lager- und Getriebeteile ist somit bei einem nach vorne beziehungsweise zur reinen Seite hin abgedichteten Druckluftmotor nicht möglich.

Ein chirurgisches Handstück der eingangs beschriebenen Art ist beispielsweise aus der US 2010/0262145 A1 bekannt. Eine Vorrichtung zum Verbinden und Schneiden ist in der WO 2009/109198 A1 beschrieben. In der US 2005/0203487 A1 ist ein Schmiersystem für ein angetriebenes chirurgisches Instrument offenbart. Und schließlich ist aus der US 2003/0000774 A1 eine Schmierkassette für ein pneumatisch angetriebenes chirurgisches Instrument bekannt.

Es ist daher eine Aufgabe der vorliegenden Erfindung, ein chirurgisches Handstück der eingangs beschriebenen Art so zu verbessern, dass der Druckluftmotor und/oder weitere Komponenten desselben einfach und sicher geschmiert werden können.

Diese Aufgabe wird bei einem chirurgischen Handstück der eingangs beschriebenen Art erfindungsgemäß dadurch gelöst, dass die mindestens eine Schmiermittelsprayverbindungsleitung seitlich am Antrieb vorbei angeordnet oder ausgebildet ist.

Ein chirurgisches Handstück der eingangs beschriebenen Art derart weiterzubilden, hat insbesondere den Vorteil, dass durch Vorsehen mindestens eines Schmiermittelsprayanschlusses der Druckluftmotor und/oder das mindestens eine Lager- oder Getriebeteil zum Schmieren derselben auf einfache Weise mit einem Schmiermittelspray beaufschlagbar sind. Hierzu muss zum Beispiel lediglich die gewünschte Schmiermittelsprayquelle mit dem Schmiermittelsprayanschluss des Handstücks gekoppelt werden. So kann auch bei einem chirurgischen Handstück, welches in integrierter Bauweise ausgebildet ist und somit den Antrieb nicht abkoppelbar umfasst, ein geeignetes Schmiermittelspray mit einem darin enthaltenen Schmiermittel, beispielsweise einem ÖI, genau dorthin geleitet werden, wo es benötigt wird, nämlich zum mindestens einen Lager- oder Getriebeteil und/oder zum Druckluftmotor. So kann eine optimale Reinigbarkeit mit einer im Vergleich zu herkömmlichen Handstücken deutlich verbesserten Schmierbarkeit von zu schmierenden Komponenten erreicht werden. Denkbar ist es, zwei, drei oder mehr Schmiermittelsprayanschlüsse am Handstück vorzusehen. Günstig ist es, wenn jedoch nur ein einziger Schmiermittelsprayanschluss vorgesehen ist. Dies ermöglicht es insbesondere, im Prinzip mit nur einem einzigen Schmiermittelspraysprühstoß die vom Handstück umfassten, zu schmierenden Komponenten zu schmieren. Günstig ist es, dass mindestens eine Schmiermittelsprayverbindungsleitung zum Ausbilden einer Fluidverbindung wischen dem mindestens einen Schmiermittelsprayanschluss und dem mindestens einen Lager- oder Getriebeteil vorgesehen ist. So kann ein Schmiermittelspray durch die Schmiermittelsprayverbindungsleitung direkt vom Schmiermittelsprayanschluss zum mindestens einen Lageroder Getriebeteil beziehungsweise auch zum Druckluftmotor gelangen, um die genannten Komponenten zu schmieren. Die Handhabung des Handstücks wird auf einfache Weise insbesondere dadurch verbessert, dass der mindestens eine Schmiermittelsprayanschluss proximalseitig des Antriebs angeordnet oder ausgebildet ist und dass die mindestens eine Schmiermittelsprayverbindungsleitung seitlich am Antrieb vorbei angeordnet oder ausgebildet ist. Diese Ausgestaltung ermöglicht es, beispielsweise von einem proximalen Ende des Handstücks her dieses zu schmieren, beispielsweise durch Beaufschlagen mit einem Schmiermittelspray, wobei die vorgeschlagene Anordnung der Schmiermittelspräyverbindungsleitung auch ein Schmieren von distal- beziehungsweise abtriebsseitig des Antriebs angeordneten Lager- oder Getriebeteilen auf einfache oder sichere Weise ermöglicht.

Vorteil ist es, wenn das Gehäuse mindestens eine Aufnahme für den mindestens einen Lager- oder Getriebeteil umfasst, in welcher Aufnahme der mindestens eine Lager- oder Getriebeteil angeordnet ist, und wenn der mindestens eine Schmiermittelsprayanschluss mit der mindestens einen Aufnahme in Fluidverbindung steht. Das Gehäuse derart auszubilden und den mindestens einen Schmiermittelsprayanschluss derart anzuordnen beziehungsweise auszubilden, gestattet es auf einfache und sichere Weise, ein Schmiermittel vom Schmiermittelsprayanschluss zum mindestens einen Lager- oder Getriebeteil zu leiten.

Günstigerweise verbindet die mindestens eine Schmiermittelsprayverbindungsleitung den mindestens einen Schmiermittelsprayanschluss und die mindestens eine Aufnahme miteinander zur Ausbildung einer Fluidverbindung. Auf diese Weise kann die gewünschte Fluidverbindung besonders einfach und sicher hergestellt werden.

Besonders ei.nfach herstellen lässt sich das Handstück, wenn die mindestens eine Schmiermittelsprayverbindungsleitung in Form einer schlauch- oder rohrförmigen Verbindungsleitung oder in Form eines am Gehäuse angeordneten oder ausgebildeten Verbindungskanals ausgebildet ist. Je nach Größe des Handstücks kann so die mindestens eine Schmiermittelsprayverbindungsleitung durch ein separates Bauteil oder durch eine entsprechende Formgebung von das Gehäuse ausbildenden Teilen im Zusammenwirken mit im Gehäuse angeordneten Komponenten ausgebildet werden. Selbstverständlich können auch zwei, drei oder mehr Schmiermittelsprayverbindungsleitungen vorgesehen sein, um einen oder mehrere Schmiermittelsprayanschlüsse mit sämtlichen zu schmierenden Komponenten des Handstücks fluidisch zu verbinden.

Günstigerweise umfasst das Gehäuse eine Antriebsaufnahme, in welcher der Antrieb angeordnet ist. So kann der Antrieb in definierter Weise am Handstück gelagert werden.

Vorzugsweise ist die mindestens eine Aufnahme abtriebsseitig der Antriebsaufnahme angeordnet oder ausgebildet. Dies ermöglicht es, beispielsweise ein Getriebe zwischen dem Antrieb und einem mit einem distalen Ende des Handstücks koppelbaren Werkzeug anzuordnen oder auszubilden.

Um Druckluftverlust am Handstück zu vermeiden, ist es vorteilhaft, wenn der Antrieb abtriebsseitig luftdicht abgedichtet im Gehäuse angeordnet ist.

Die Handhabbarkeit des Handstücks insbesondere beim Schmieren desselben kann insbesondere dadurch verbessert werden, dass das Handstück nur einen einzigen Schmiermittelsprayanschluss umfasst. Beispielsweise kann so mit nur einem oder auch zwei oder mehr Sprühstößen Schmiermittel in Form eines Schmiermittelsprays in das Handstück eingebracht werden zum Schmieren der eine Schmierung benötigenden Komponenten.

Besonders kompakt ausbilden lässt sich das Handstück, wenn der mindestens eine Schmiermittelsprayanschluss in einer Gehäusewand des Gehäuses und/oder von der Gehäusewand abstehend angeordnet oder ausgebildet ist. So lässt sich der Schmiermittelsprayanschluss auf einfache Weise in das Handstück integrieren.

Um eine Zugänglichkeit des mindestens einen Lager- oder Getriebeteils zu verbessern, wenn dieses abtriebsseitig bezogen auf den Antrieb am Handstück angeordnet oder ausgebildet ist, ist es günstig, wenn der mindestens eine Schmiermittelsprayanschluss abtriebsseitig des Antriebs angeordnet oder ausgebildet ist. Auf diese Weise kann das Schmiermittel auf möglichst kurzem Weg vom Schmiermittelsprayanschluss zum mindestens einen Lager- oder Getriebeteil geleitet werden.

Die Zugänglichkeit des Schmiermittelsprayanschlusses lässt sich auf einfache Weise dadurch verbessern, dass der mindestens eine Schmiermittelsprayanschluss an einem proximalen Ende des Handstücks angeordnet oder ausgebildet ist. So kann eine Schmierung des Handstücks wie eingangs beschrieben auf einfache Weise von der reinen zur unreinen Seite hin erfolgen.

Um eine möglichst schlanke Bauform des Handstücks zu ermöglichen, ist es günstig, wenn der mindestens eine Schmiermittelsprayanschluss in proximaler Richtung weisend angeordnet oder ausgebildet ist.

Gemäß einer weiteren bevorzugten Ausführungsform der Erfindung kann vorgesehen sein, dass der mindestens eine Schmiermittelsprayanschluss ein selbstschließendes Einlassventil umfasst. Auf diese Weise kann sichergestellt werden, dass der mindestens eine Schmiermittelsprayanschluss verschlossen ist, wenn er nicht mit einem Schmiermittelspray beaufschlagt wird, welches unter einem Mindestdruck steht, um das Einlassventil zu öffnen. So kann verhindert werden, dass Verunreinigungen durch den Schmiermittelsprayanschluss ins Innere des Handstücks gelangen können und umgekehrt Schmiermittel austreten kann.

Um den Druckluftmotor auf einfache und sichere Weise betreiben zu können, ist es vorteilhaft, wenn das Handstück mindestens einen Drucklufteinlass und mindestens einen Druckluftauslass umfasst, welche mit dem Druckluftmotor in Fluidverbindung stehen. Durch den mindestens einen Drucklufteinlass kann der Druckluftmotor mit Druckluft beaufschlagt werden, die dann aus dem Handstück wieder durch den mindestens einen Druckluftauslass herausströmen kann. Denkbar sind jeweils zwei, drei, vier oder noch mehr Drucklufteinbeziehungsweise Auslässe.

Ein besonders einfacher und kompakter Aufbau des Handstücks wird insbesondere möglich, wenn der mindestens eine Drucklufteinlass den mindestens einen Schmiermittelsprayanschluss bildet. Mit anderen Worten kann das Schmiermittel direkt durch den mindestens einen Drucklufteinlass ins Innere des Handstücks geleitet werden.

Ferner kann es vorteilhaft sein, wenn der mindestens eine Druckluftauslass den mindestens einen Schmiermittelsprayanschluss bildet. Auf diese Weise kann ein Schmiermittel insbesondere durch den Druckluftauslass ins Innere des Handstücks geleitet werden, beispielsweise um den Druckluftmotor zu schmieren. Optional kann auch ein Ventil vorgesehen sein, welches den Druckluftauslass in einer Betriebsstellung von der Schmiermittelsprayverbindungsleitung trennt und sich erst unter Druckbeaufschlagung in einer Schmierstellung, beispielsweise beim Einleiten eines Schmiermittelsprays, öffnet, um so auch das mindestens eine Lager- oder Getriebeteil zu schmieren.

Gemäß einer weiteren bevorzugten Ausführungsform der Erfindung kann vorgesehen sein, dass der Druckluftmotor eine Rotorwelle umfasst und dass die Rotorwelle die mindestens eine Schmiermittelsprayverbindungsleitung umfasst. Diese Ausgestaltung ermöglicht es, ein Schmiermittel durch die Rotorwelle hindurch zu einem abtriebsseitig angeordneten Lager- oder Getriebeteil zu leiten.

Vorzugsweise ist die Rotorwelle in Form eines hohlen Schafts ausgebildet ist, welcher einen Kanal definiert. Dies gestattet es, das Schmiermittel direkt durch die Rotorwelle-hindurch zu leiten.

Besonders kompakt ausbilden lässt sich das chirurgische Handstück, wenn der Kanal die mindestens eine Schmiermittelsprayverbindungsleitung bildet. Mit anderen Worten kann so die Rotorwelle selbst die Schmiermittelsprayverbindungsleitung bilden.

Günstig kann es ferner sein, wenn die mindestens eine Schmiermittelsprayverbindungsleitung im Kanal angeordnet und relativ zum Gehäuse feststehend ausgebildet ist. Auf diese Weise kann verhindert werden, dass die Rotorwelle im Inneren mit einem Schmiermittel beaufschlagt wird, welches vorzugsweise direkt zum mindestens einen Lager- oder Getriebeteil geleitet werden soll.

Besonders kompakt ausbilden lassen sich das Handstück und insbesondere dessen Druckluftanschlüsse, wenn der mindestens eine Drucklufteinlass koaxial zur Rotorwelle des Druckluftmotors angeordnet oder ausgebildet ist.

Ferner kann es vorteilhaft sein, wenn der mindestens eine Druckluftauslass koaxial zur Rotorwelle des Druckluftmotors angeordnet oder ausgebildet ist. Eine koaxiale Ausbildung eines Drucklufteinlasses oder eines Druckluftauslasses ermöglicht es insbesondere, mit nur jeweils einem einzigen Einlass beziehungsweise Auslass am Handstück auszukommen.

Gemäß einer weiteren bevorzugten Ausführungsform der Erfindung kann vorgesehen sein, dass in oder an der mindestens einen Schmiermittelsprayverbindungsleitung ein in einer Schließstellung druckluftdichtes Schließelement angeordnet oder ausgebildet ist. Mit dem Schließelement kann insbesondere die Schmiermittelsprayverbindungsleitung geschlossen werden, wenn das Handstück im Einsatz ist, also insbesondere der Antrieb mit Druckluft beaufschlagt wird. So kann vermieden werden, dass Druckluft in unerwünschter Weise durch die Schmiermittelsprayverbindungsleitung entweicht.

Die Handhabung des chirurgischen Handstücks wird besonders einfach, wenn das Schließelement in Form eines selbstschließenden Ventils ausgebildet ist, welches in einer Schmierstellung offenbar ist. Beispielsweise kann es derart angeordnet und ausgebildet sein, dass es selbsttätig öffnet, wenn ein Schmiermittelspray durch den Schmiermittelsprayanschluss in das Handstück eingeleitet wird, beispielsweise wenn der im Schmiermittelspray herrschende Druck hierfür so groß ist, dass das Ventil automatisch öffnet. Dann kann das Schmiermittel durch die Schmiermittelsprayverbindungsleitung hindurchströmen und zu schmierende Komponenten des Handstücks erreichen.

Um zu vermeiden, dass Verunreinigungen ins Innere des Handstücks gelangen können oder Druckluft, die zum Antreiben des Druckluftmotors vorgesehen ist, in unerwünschter Weise entweichen kann, ist es vorteilhaft, wenn das Handstück mindestens einen Schmiermittelsprayanschlussverschluss zum Verschließen des mindestens einen Schmiermittelsprayanschlusses umfasst.

Auf besonders einfache Weise lässt sich der mindestens eine Schmiermittelsprayanschlussverschluss ausbilden, wenn er an einer mit dem Handstück koppelbaren Druckluftkupplung angeordnet oder ausgebildet ist. Dies bedeutet, dass beispielsweise, wenn das Handstück mit der Druckluftkupplung gekoppelt ist, die wiederum mit einer Druckluftquelle verbunden ist, der Schmiermittelsprayanschluss durch den Schmiermittelsprayanschlussverschluss automatisch verschlossen wird. Eine Bedienperson verschließt also den Schmiermittelsprayanschluss automatisch dadurch, dass sie das Handstück mit einer hierfür entsprechend ausgebildeten Druckluftkupplung in Eingriff bringt.

Günstig kann es ferner sein, wenn das Handstück ein selbstschließendes Abluftkanalschließventil umfasst, welches in einer Betriebsstellung geschlossen und in einer Schmierstellung geöffnet ist zum Ausbilden einer Fluidverbindung zwischen einem mit dem mindestens einen Drucklufteinlass und dem Druckluftmotor verbundenen Abluftkanal und der mindestens einen Schmiermittelsprayverbindungsleitung. Das Abluftkanalschließventil ermöglicht es also insbesondere, entweder eine Fluidverbindung zwischen dem mindestens einen Druckluftauslass und der Schmiermittelsprayverbindungsleitung herzustellen oder eben nicht. Zum Schmieren des Handstücks ist eine solche Fluidverbindung gewünscht, zum Betreiben des Handstücks mit Druckluft jedoch nicht.

Des Weiteren kann es günstig sein, wenn das Handstück eine Druckluftzufuhrunterbrechungseinrichtung umfasst, welche in einer Betriebsstellung geöffnet ist zum Ausbilden einer Fluidverbindung zwischen einem mit dem mindestens einen Drucklufteinlass und dem Druckluftmotor verbundenen Zuluftkanal und welche in einer Schmierstellung geschlossen ist zum Unterbrechen der Fluidverbindung zwischen dem mindestens einen Drucklufteinlass und dem Zuluftkanal. Die Druckluftzufuhrunterbrechungseinrichtung ermöglicht somit das wahlweise Unterbrechen einer Fluidverbindung zwischen dem mindestens einen Drucklufteinlass und dem Zuluftkanal. So kann beispielsweise erreicht werden, dass Schmiermittel nicht durch den Zuluftkanal zum Druckluftmotor gelangen kann, wenn dies nicht gewünscht ist.

Um das mindestens eine Lager- oder Getriebeteil einfach und sicher mit Schmiermittel beaufschlagen zu können, ist es vorteilhaft, wenn die mindestens eine Schmiermittelsprayverbindungsleitung in der Schmierstellung geöffnet ist.

Besonders vorteilhaft ist es, wenn die Druckluftzufuhrunterbrechungseinrichtung ein am Gehäuse bewegbar gehaltenes Schließelement oder ein Ventil oder einen Teil einer mit dem Handstück koppelbaren Druckluftkupplung umfasst. Auf diese Weise kann insbesondere eine Fluidverbindung zwischen dem mindestens einen Drucklufteinlass und dem Zuluftkanal auf einfache Weise, und zwar beispielsweise manuell von einer Bedienperson betätigt oder automatisch, durch ein entsprechend ausgebildetes Ventil unterbrochen werden. Vorzugweise ist das Schließelement manuell betätigbar ausgebildet ist. Dies gestattet es einer Bedienperson, die Fluidverbindung zwischen dem Drucklufteinlass und dem Zuluftkanal in gewünschter Weise zu öffnen und zu schließen.

Gemäß einer bevorzugten Ausführungsform der Erfindung kann vorgesehen sein, dass das mindestens eine Lager- oder Getriebeteil in Form eines Kugellagers, eines Gleitlagers, eines Nadellagers, einer Lagerwelle oder eines Zahnrads ausgebildet ist. Selbstverständlich können auch zwei, drei oder mehr Getriebeteile am Handstück vorgesehen sein, die durch Beaufschlagen mit einem Schmiermittelspray geschmiert werden können.

Um mit dem Handstück gewünschte chirurgische Eingriffe vornehmen zu können ist es vorteilhaft, wenn es ein mit dem Handstück distalseitig gekoppeltes chirurgisches Werkzeug umfasst. Das Werkzeug kann wahlweise mit dem Handstück lösbar verbindbar oder auch dauerhaft fest mit diesem verbunden ausgebildet sein.

Vorzugsweise ist das Werkzeug ein Bohr-, Fräs-, Säge-, Schraub- oder Schneidwerkzeug. Mit einem solchen Werkzeug lassen sich eine Vielzahl von chirurgischen Eingriffen in gewünschter Weise durchführen.

Die nachfofgende Beschreibung bevorzugter Ausführungsformen der Erfindung und von ßeispielen chirurgischer Handstücke dient im Zusammenhang mit den Zeichnungen der näheren Erläuterung. Es zeigen:
- Figur 1:: eine schematische Längsschnittansicht eines ersten Beispiels eines chirurgischen Handstücks;
- Figur 1A:: eine vergrößerte Ansicht des Ausschnitts A in Figur 1;
- Figur 2:: eine Schnittansicht analog Figur 1 eines erfindungsgemäßen Ausführungsbeispiels eines chirurgischen Handstücks;
- Figur 2A:: eine vergrößerte Ansicht des Bereichs B in Figur 2;
- Figur 3:: eine Schnittansicht analog Figur 1 eines weiteren Beispiels eines chirurgischen Handstücks;
- Figur 4:: eine Schnittansicht analog Figur 1 eines weiteren Beispiels eines chirurgischen Handstücks;
- Figur 4A:: eine Ansicht in Richtung des Pfeils C in Figur 4;
- Figur 5:: eine Schnittansicht analog Figur 1 eines weiteren Beispiels eines chirurgischen Handstücks;
- Figur 6:: eine Schnittansicht analog Figur 1 eines weiteren Beispiels eines chirurgischen Handstücks;
- Figur 7:: eine Schnittansicht analog Figur 1 eines weiteren Beispiels eines chirurgischen Handstücks;
- Figur 7A:: eine vergrößerte Ansicht des Bereichs D in Figur 7; und
- Figur 8:: eine Schnittansicht analog Figur 1 eines weiteren erfindungrgemäßen Ausführungsbeispiels eines chirurgischen Handstücks.

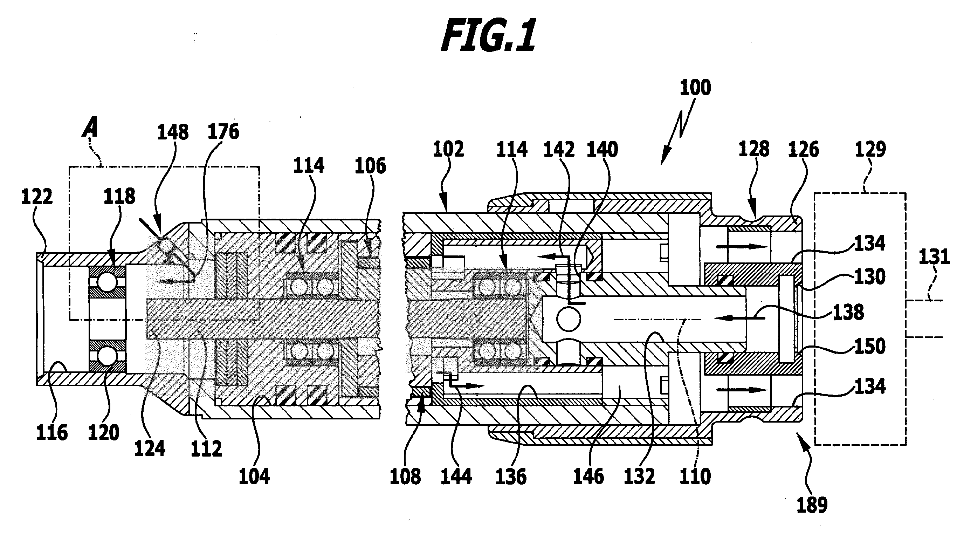

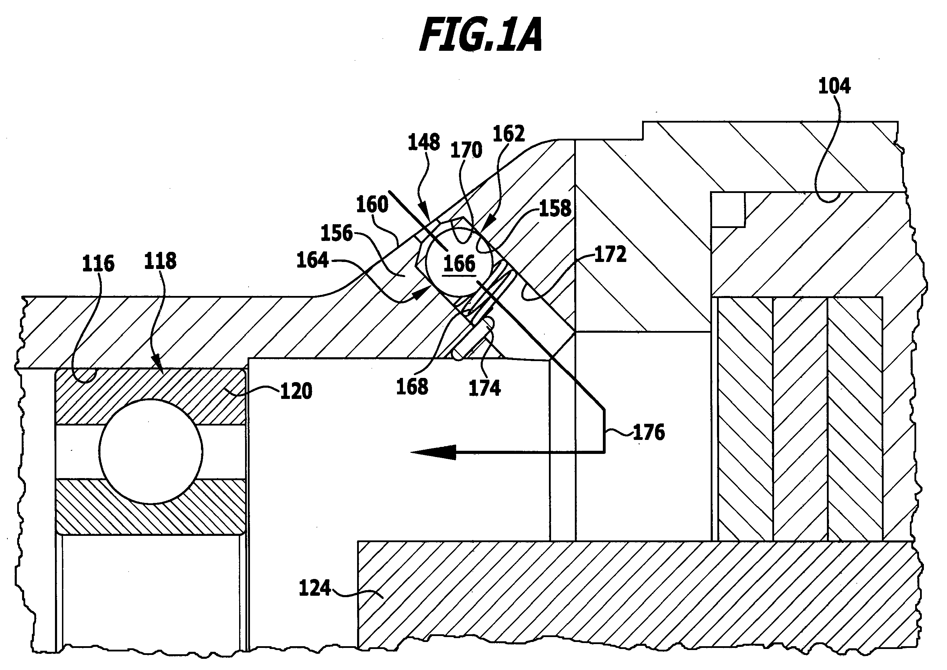

In Figur 1 ist ein erstes Ausführungsbeispiel eines chirurgischen Handstücks schematisch im Längsschnitt dargestellt und insgesamt mit dem Bezugszeichen 100 bezeichnet. Es umfasst ein Gehäuse 102 mit einer Ausnehmung in Form einer Antriebsaufnahme 104, in welcher ein Antrieb 106 in Form eines Druckluftmotors 108 angeordnet ist.

Das Handstück 100 definiert eine Längsachse 110, welche gleichzeitig eine Längsachse einer Rotorwelle 112 des Druckluftmotors 108 bildet. Die Rotorwelle 112 ist an ihrem proximalen Ende sowie abtriebsseitig des Antriebs 106 jeweils mit einem Kugellager 114 am Gehäuse 102 gelagert. Ferner ist der Antrieb 106 distal- oder abtriebsseitig fluiddicht im Gehäuse 102 angeordnet. Die Rotorwelle 112 ragt in eine Aufnahme 116 für ein Lager- oder Getriebeteil 118 hinein, welches beispielsweise in Form eines in Figur 1 schematisch dargestellten Kugellagers 120 ausgebildet sein kann. Ein distales Ende 122 des Gehäuses 102 ist offen, so dass ein proximales Ende eines in den Figuren nicht dargestellten Bearbeitungswerkzeugs in die Aufnahme 116 eingeführt und mit einem distalen Ende 124 der Rotorwelle 112 gekoppelt werden kann. Ein Schaft des Werkzeugs kann sich dann beispielsweise über das Kugellager 120 am Gehäuse 102 abstützen und durch das Kugellager 120 koaxial zur Längsachse 110 ausgerichtet werden.

Ein proximales Ende 126 des Gehäuses 102 ist vorzugsweise in Form eines Kupplungsstücks 128 ausgebildet zum lösbaren Verbinden, das heißt temporären Koppeln, mit einer Schlauchkupplung, welche an einem Ende eines in den Figuren nicht dargestellten Verbindungsschlauchs zum Verbinden des Handstücks 100 mit einer nicht dargestellten Druckluftversorgung oder Druckluftquelle ausgebildet ist. Das Kupplungsstück 128 weist einen koaxial zur Längsachse 110 ausgebildeten Drucklufteinlass 130 auf, welcher über einen Zuluftkanal 132 einlassseitig mit dem Druckluftmotor 108 in Fluidverbindung steht. Von der Längsachse 110 radial beabstandet sind am Kupplungsstück 128 mehrere in proximaler Richtung hin geöffnete, im Querschnitt kreisförmige Druckluftauslässe 134 den Drucklufteinlass 130 umgebend ausgebildet, die abluftseitig mit dem Druckluftmotor 108 über einen Abluftkanal 136 in Fluidverbindung stehen.

Zum Antreiben des Druckluftmotors 108 wird der Drucklufteinlass 130 mit Druckluft beaufschlagt, welche in Richtung des Pfeils 138 koaxial zur Längsachse 110 in das Handstück 100 hineinströmt und nach Durchströmen eines proximalseitig des Druckluftmotors 108 ausgebildeten Querkanals 140 schließlich wieder parallel zur Längsachse 110 zum Druckluftmotor 108 in distaler Richtung geleitet wird. Ein Strömungspfad der Druckluft durch den Querkanal 140 ist durch den Pfeil 142 schematisch angedeutet. Abluftseitig strömt die Druckluft in Richtung des durch den Pfeil 144 symbolisierten Strömungspfads in proximaler Richtung zurück durch den Abluftkanal 136, welcher mit einem Ringraum 146 in Fluidverbindung steht, der den Zuluftkanal 132 konzentrisch umgibt. Der Ringraum 146 steht wiederum in Fluidverbindung mit den Druckluftauslässen 134.

Zum Schmieren des Kugellagers 120 ist ein Schmiermittelsprayanschluss 148 vorgesehen, welcher abtriebsseitig des Druckluftmotors 108 in einer Wand 156 des Gehäuses 102 ausgebildet ist. Er umfasst eine Bohrung 158, welche gegenüber der Längsachse 110 um etwa 45° geneigt in distaler Richtung und von der Längsachse 110 weg weisend ausgebildet ist. Ein Neigungswinkel zwischen der Bohrung 158 und der Längsachse 110 kann wahlweise in einem Winkelbereich von 0° bis 180° liegen. Zu einer Außenseite 160 der Wand 156 hin verjüngt sich die Bohrung 158. In ihr ist ein Schließelement 162 in Form eines selbstschließenden Ventils 164 angeordnet. Es umfasst eine Kugel 166, welche durch eine Schraubenfeder 168 gegen einen konischen Ventilsitz 170 gedrückt wird. Die Schraubenfeder 168 stützt sich einerseits an der Kugel und andererseits an einem von einer Innenwand 172 der Bohrung 158 vorstehenden Vorsprungs 174.

Wird von außen an das Gehäuse 102 beispielsweise ein Kupplungsstück einer Schmiermittelsprayquelle angesetzt, kann unter Druck stehendes Schmiermittelspray die Kugel 166 entgegen der Wirkung der Schraubenfeder 168 in die Bohrung 158 hineinbewegen, so dass das Ventil 164 geöffnet und Schmiermittel durch die Bohrung 158 im Wesentlichen längs eines durch den Pfeil 176 schematisch repräsentierten Strömungspfads in die Aufnahme 116 einströmen kann zum Schmieren des Lager- oder Getriebeteils 118.

Ferner kann auch der Drucklufteinlass 130 einen Schmiermittelanschluss 150 bilden, um durch entsprechende Ankopplung einer Schmiermittelsprayquelle den Druckluftmotor 108 nach einer Reinigung und vor einer Sterilisation zu schmieren. Das Schmiermittelspray folgt dann dem oben beschriebenen Strömungspfad.

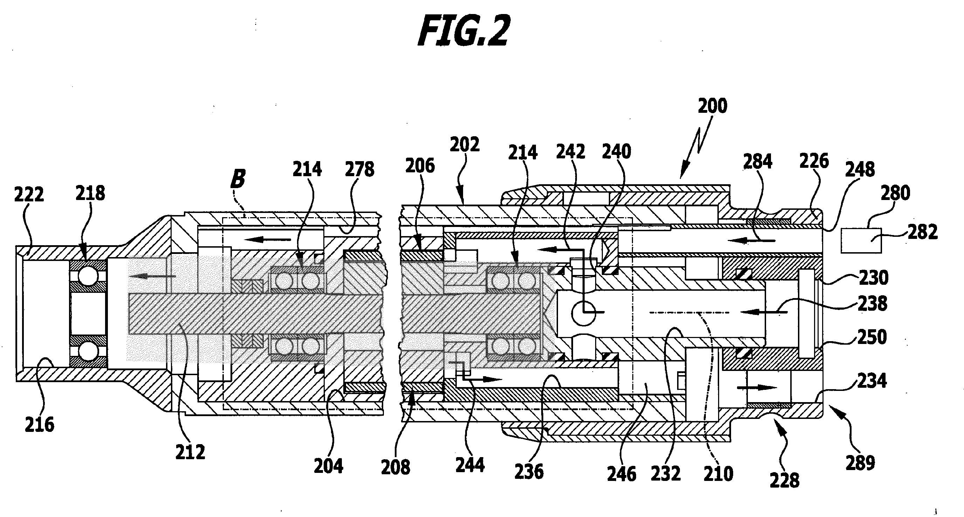

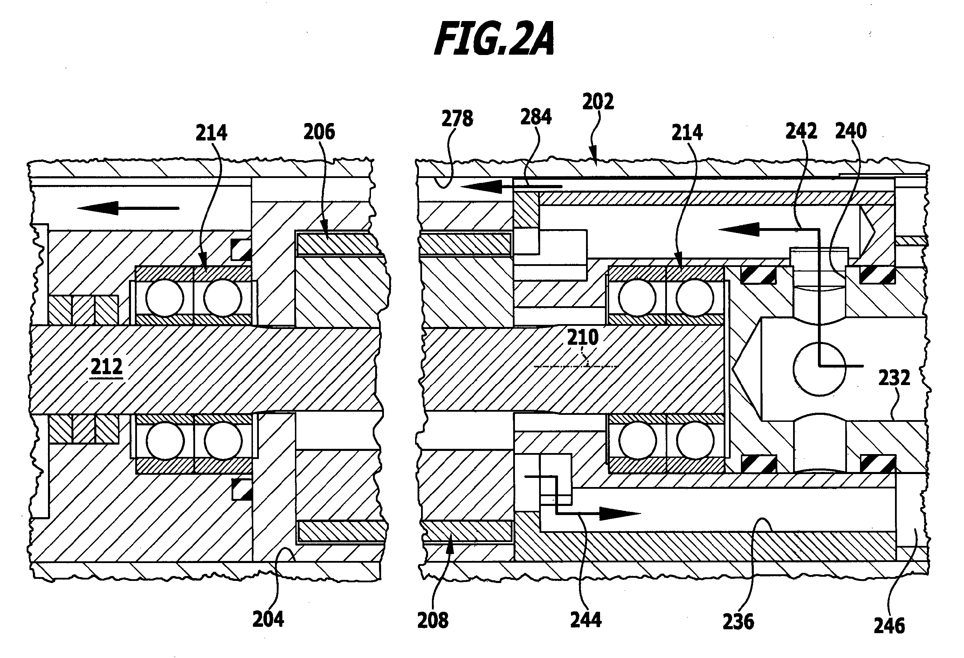

In Figur 2 ist schematisch ein weiteres Ausführungsbeispiel eines chirurgischen Handstücks dargestellt und insgesamt mit dem Bezugszeichen 200 bezeichnet. Bauteile und Komponenten des Handstücks 200, die mit denen des Handstücks 100 übereinstimmen, weisen identische, zweistellige Endziffern auf. Dies gilt im Übrigen auch für alle noch nachfolgend näher beschriebenen Ausführungsbeispiele.

Der Aufbau des Handstücks 200 unterscheidet sich vom Handstück 100 im Wesentlichen dadurch, dass der Schmiermittelanschluss 248 nicht abtriebsseitig des Antriebs 206 vorgesehen ist, sondern in proximaler Richtung weisend am Ende 226. Zur Herstellung einer Fluidverbindung zwischen dem Schmiermittelsprayanschluss 248 und der Aufnahme 216 dient eine Schmiermittelsprayverbindungsleitung 278, die exzentrisch zur Längsachse 210, im Wesentlichen jedoch parallel zu dieser verlaufend, seitlich am Antrieb 206 vorbei den Schmiermittelsprayanschluss 248 und die Aufnahme 216 fluidisch verbindet. Wird eine Schmiermittelsprayquelle 180, die schematisch in Figur 2 dargestellt ist und beispielsweise in Form einer Spraydose 282 ausgebildet sein kann, mit dem Schmiermittelsprayanschluss 248 gekoppelt, so kann das Schmiermittelspray in Richtung des Pfeils 284 in die Schmiermittelsprayverbindungsleitung 278 ein- und seitlich am.Antrieb vorbei in die Aufnahme 216 strömen und das Kugellager 220 schmieren. Zum Schmieren des Druckluftmotors 208 kann der Drucklufteinlass 230 als Schmiermittelsprayanschluss 250 genutzt werden, und zwar in der oben beschriebenen Weise.

Das Handstück 200 ermöglicht somit eine einfache Schmierung des Lager- oder Getriebeteils 118 von der reinen Seite des Handstücks 200 aus zur unreinen Seite hin, also zum distalen Ende 222, welches mit einem Bearbeitungswerkzeug gekoppelt werden kann.

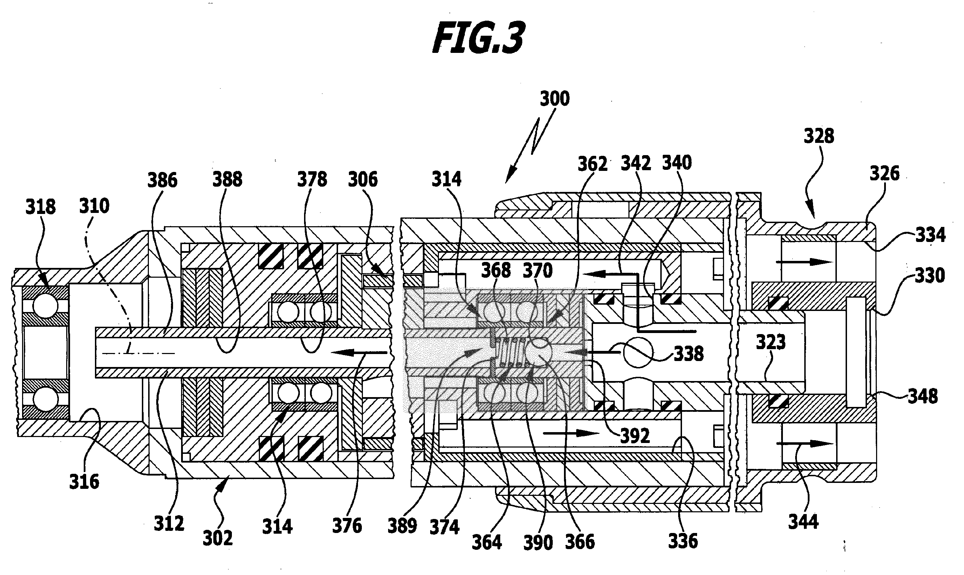

Ein weiteres Ausführungsbeispiel eines chirurgischen Handstücks ist in Figur 3 schematisch dargestellt und insgesamt mit dem Bezugszeichen 300 bezeichnet. Wie bereits beim Handstück 200 angegeben sind mit Komponenten und Beuteilen des Handstücks 100 identische Bauteile und Komponenten des Handstücks 300 mit denselben zweistelligen Endziffern bezeichnet.

Der Aufbau des Handstücks 300 entspricht im Wesentlichen dem des Handstücks 100. Er unterscheidet sich jedoch insbesondere dadurch, dass die Rotorwelle 312 in Form eines hohlen Schafts 386 ausgebildet ist, welche in ihrem Inneren einen Kanal 388 definiert, welcher eine Schmiermittelsprayverbindungsleitung 378 bildet. Proximalseitig ist der Kanal 388 durch eine Druckluftzufuhrunterbrechungseinrichtung 389 verschlossen, welche ein Schließelement 390 in Form eines Ventils 364 umfasst. Das Ventil 364 umfasst eine Kugel 366, welche durch eine Schraubenfeder 368 vorgespannt ist, welche sich distalseitig an einem ringförmigen Vorsprung 374 des Kanals 388 abstützt und andererseits an der Kugel 366, so dass diese gegen einen in distaler Richtung weisenden hohlkugeligen Ventilsitz 370 gedrückt wird, um in einer in Figur 3 dargestellten Schließstellung den Kanal 388 zu verschließen.

Beim Handstück 300 bildet der Drucklufteinlass 330 den einzigen Schmiermittelsprayanschluss 348. Durch ihn hindurch kann beispielsweise ein Anschlussrohr einer Schmiermittelsprayquelle mit einer Einlassöffnung 392 des Kanals 388 gekoppelt werden, so dass das Ventil 364 unter Druckbeaufschlagung beim Einleiten eines Schmiermittelsprays öffnet und Schmiermittel in Richtung des Pfeils 376 koaxial zur Längsachse 310 in die Aufnahme 316 strömen kann zum Schmieren des Kugellagers 320. Aufgrund seines Aufbaus ist das Ventil 364 selbsttätig schließend ausgebildet, wenn es nicht durch einen entsprechenden Schmiermittelspraydruck geöffnet wird. Vorzugsweise ist das Ventil 364 so gestaltet, dass es bei einem Betrieb des Handstücks 300, also unter Druckluftbeaufschlagung des Druckluftmotors 308, noch geschlossen ist, also in der in Figur 3 dargestellten Schließstellung die Schmiermittelsprayverbindungsleitung 378 druckluftdicht verschließt. Ist das Ventil 364 geöffnet, nimmt es eine Schmierstellung ein. Wie bei den Handstücken 100 und 200 wird durch Einleiten eines Schmiermittelsprays durch den Drucklufteinlass 330 auch der Druckluftmotor 308 geschmiert.

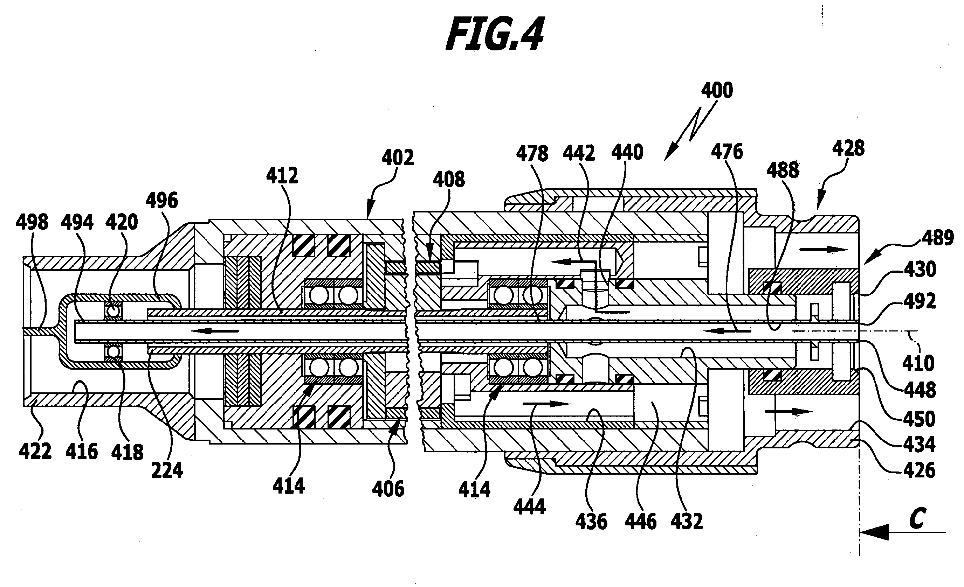

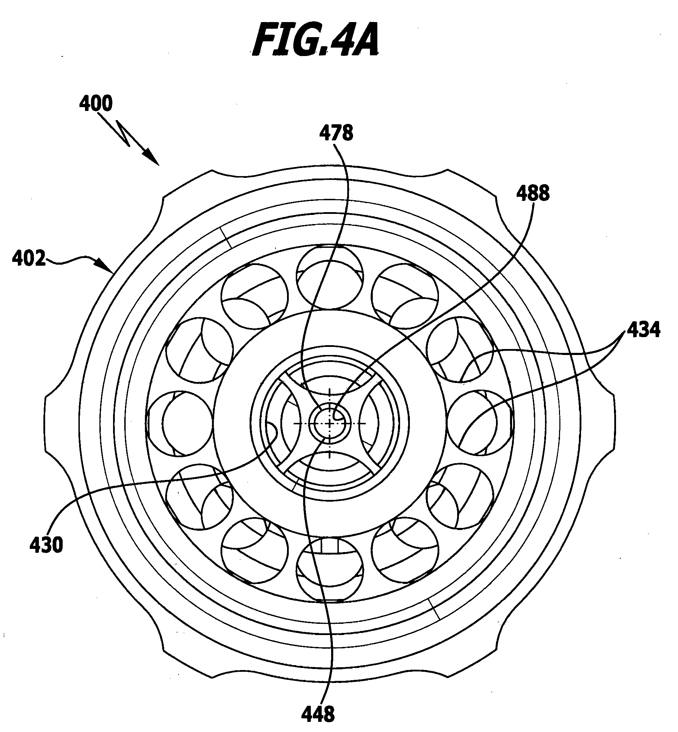

In Figur 4 ist ein weiteres Ausführungsbeispiel eines chirurgischen Handstücks schematisch dargestellt und insgesamt mit dem Bezugszeichen 400 bezeichnet. Es ist in seinem Aufbau dem Handstück 300 ähnlich.

Auch das Handstück 400 weist eine Rotorwelle 412 auf, die einen sich koaxial zur Längsachse 410 erstreckenden Kanal 488 definiert. Im Kanal 488 ist eine sich bis zum proximalen Ende 426 des Handstücks 400 erstreckende rohrförmige Schmiermittelsprayverbindungsleitung 478 angeordnet, welche die Rotorwelle 412 jedoch nicht berührt und relativ zum Gehäuse 402 feststehende angeordnet ist. Im Bereich eines distales Endes 494 der Schmiermittelsprayverbindungsleitung 478 trägt diese außen ein Lager- oder Getriebeteil 418 in Form eines Kugellagers 420. Radial außen stützt sich das Kugellager 420 an einer Kupplungshülse 496 ab, die proximalseitig ein distales Ende 424 der Rotorwelle 412 übergreift und drehfest mit dieser verbunden ist. Die Kupplungshülse 496 umfasst einen in distaler Richtung weisenden Kupplungsvorsprung 498, welcher mit einem Werkzeug in Eingriff gebracht werden kann, um dieses in Drehung zu versetzen. Ein proximales Ende der Schmiermittelsprayverbindungsleitung 478 definiert eine Einlassöffnung 492 in Form eines Schmiermittelsprayanschlusses 448. Dieser ist koaxial umgeben von einem Drucklufteinlass 430.

Zum Schmieren des Kugellagers 420 wird eine Schmiermittelsprayquelle mit dem Schmiermittelsprayanschluss 448 gekoppelt und das Schmiermittelspray koaxial zur Längsachse 410 in Richtung des Pfeils 476 durch die den Kanal 488 durchsetzende Schmiermittelsprayverbindungsleitung 478 geleitet zum Schmieren des Kugellagers 420. Mit Schmiermittelspray, welches durch den Drucklufteinlass 430 eingeleitet wird, der wiederum einen Schmiermittelsprayanschtuss 450 bildet, kann der Druckluftmotor 408 geschmiert werden.

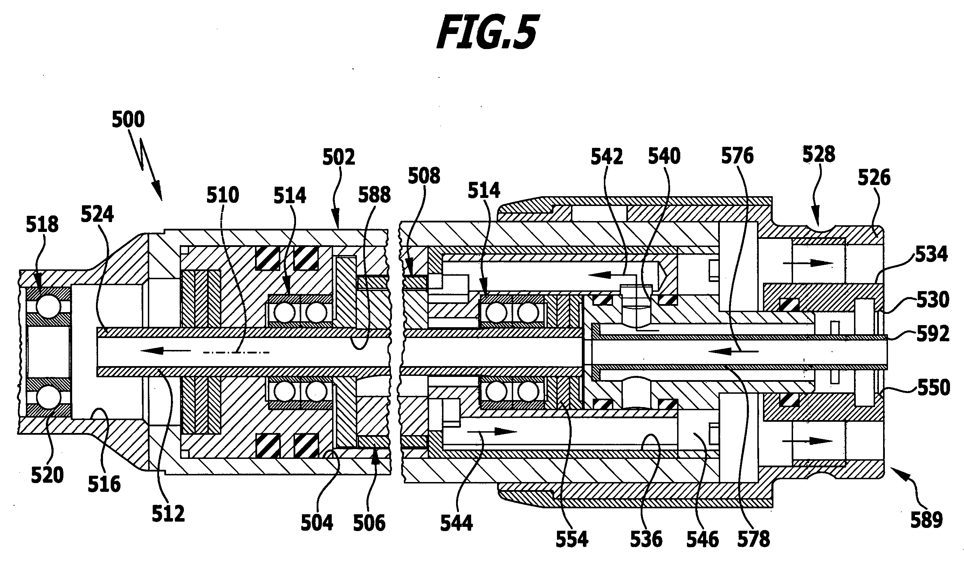

In Figur 5 ist schematisch ein weiteres Ausführungsbeispiel eines insgesamt mit dem Bezugszeichen 500 bezeichneten chirurgischen Handstücks dargestellt. Es ist im Aufbau dem chirurgischen Handstück 400 ähnlich.

Eine rohrförmige Schmiermittelsprayverbindungsleitung 578 erstreckt sich von einem proximalen Ende 526 koaxial zur Längsachse 510 bis nahe an ein proximales Ende der Rotorwelle 512 heran. Die Schmiermittelsprayverbindungsleitung 578 ist relativ zum Gehäuse 502 feststehend ausgebildet. Sie ist wiederum koaxial umgeben von einem Drucklufteinlass 530, welcher einen Schmiermittelsprayanschluss 550 bildet. Die Rotorwelle 512 ist proximalseitig, also in einem Bereich ihres proximalen Endes, von ringförmigen Dichtelementen 554 umgeben, welche die Rotorwelle 512 fluiddicht gegenüber der Schmiermittelsprayverbindungsleitung 578 abdichten.

In der Aufnahme 516 ist ein Kugellager 520 wie beim Handstück 100 angeordnet. Zum Schmieren desselben kann durch eine in proximaler Richtung weisende Einlassöffnung 592 der Schmiermittelsprayverbindungsleitung 578 ein Schmiermittelspray eingeleitet und in Richtung des Pfeils 576 durch die Schmiermittelsprayverbindungsleitung 578 hindurch in den Kanal 588 der schaftförmigen Rotorwelle 512 und durch diesen hindurch bis zur Aufnahme 516 geleitet werden, um das Kugellager 520 zu schmieren. Zur Schmierung des Druckluftmotors 508 dient der Schmiermittelsprayanschluss 550.

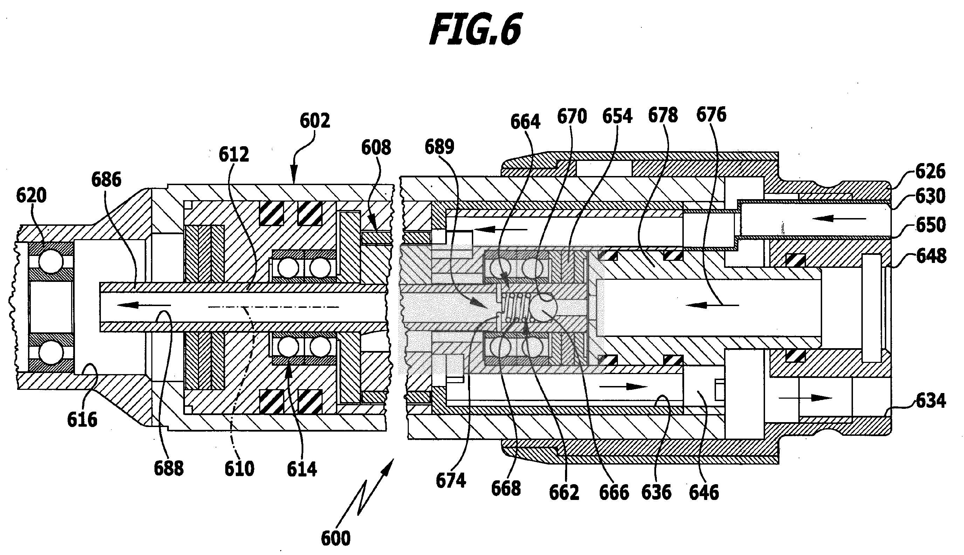

In Figur 6 ist schematisch ein weiteres Ausführungsbeispiel eines chirurgischen Handstücks dargestellt und insgesamt mit dem Bezugszeichen 600 bezeichnet. Es hat Ähnlichkeit mit dem Handstück 500, unterscheidet sich jedoch von diesem insbesondere dadurch, dass der Drucklufteinlass 630 seitlich versetzt zur Längsachse 610 am proximalen Ende 626 ausgebildet ist.

Ebenfalls seitlich versetzt zur Längsachse 610 sind mehrere Druckluftauslässe 634 vorgesehen, die mit dem Ringraum 646 gekoppelt sind. Koaxial zur Längsachse 610 ist in proximaler Richtung weisend ein Schmiermittelsprayanschluss 648 ausgebildet, welcher mit einer relativ zum Gehäuse 602 feststehenden Schmiermittelsprayverbindungsleitung 678 in Fluidverbindung steht. Die Rotorwelle 612 ist wie beim Handstück 500 in Form eines Schafts 686 ausgebildet und mittels Dichtelementen 654 gegenüber der Schmiermittelsprayverbindungsleitung 678 proximalseitig abgedichtet. Der Kanal 688 bildet quasi eine Fortsetzung der Schmiermittelsprayverbindungsleitung 678, so dass in dieser, wie beim Handstück 300, ein Schließelement 662 in Form eines Ventils 664 in der Rotorwelle 612 angeordnet ist, um den Kanal 688 selbsttätig zu verschließen. Das Ventil umfasst eine Kugel 666, welche von einer Schraubenfeder 668, die sich an einem distalseitig angeordneten ringförmigen Vorsprung 674 des Kanals 688 abstützt, in proximaler Richtung gegen einen Ventilsitz 670 gedrückt wird, um in der in Figur 6 dargestellten Schließstellung den Kanal 688 zu verschließen.

Wird der Schmiermittelsprayanschluss 648 mit einer Schmiermittelsprayquelle gekoppelt, öffnet sich das Ventil 664 aufgrund des wirkenden Drucks und ermöglicht das Einströmen eines Schmiermittelsprays durch die Schmiermittelsprayverbindungsleitung 678 koaxial zur Längsachse in Richtung des Pfeils 676 durch das in der Schmierstellung geöffnete Ventil 664 sowie den Kanal 688 hindurch in die Aufnahme 616 zum Schmieren des Kugellagers 620. Zum Schmieren des Druckluftmotors 608 kann der Drucklufteinlass 630 als Schmiermittelsprayanschluss 650 genutzt werden.

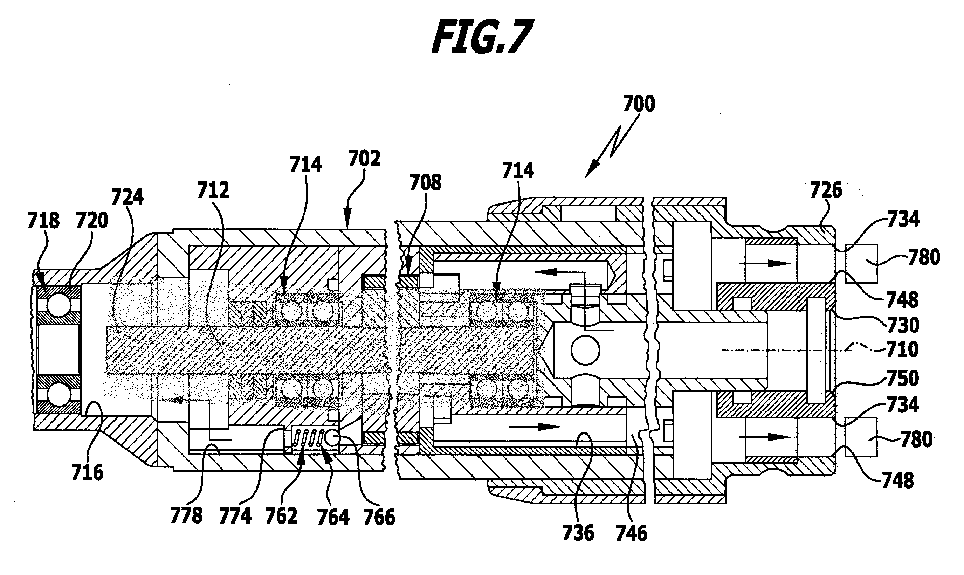

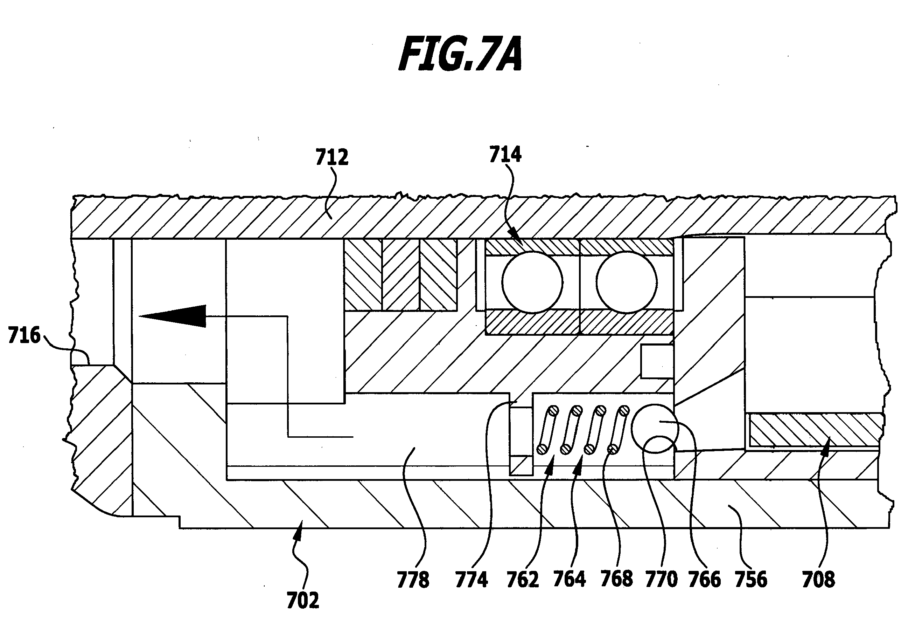

In Figur 7 ist schematisch ein weiteres Ausführungsbeispiel eines chirurgischen Handstücks dargestellt und insgesamt mit dem Bezugszeichen 700 bezeichnet. Es ist in seinem Aufbau dem Handstück 100 am ähnlichsten. Es unterscheidet sich jedoch vom Handstück 100 dadurch, dass die Druckluftauslässe 734 als Schmiermittelsprayanschlüsse 748 genutzt werden können.

Abtriebseitig des Druckluftmotors 708 ist parallel zur Längsachse 710 und nahe einer äußeren Wand 756 des Gehäuses 702 eine kanalartige Schmiermittelsprayverbindungsleitung 778 ausgebildet zur Herstellung einer Fluidverbindung zwischen den Druckluftauslässen 734 und der Aufnahme 716. In der Schmiermittelsprayverbindungsleitung 778 ist ein Schließelement 762 in Form eines Ventils 764 angeordnet. Das Ventil 764 umfasst eine Kugel 766, welche durch eine Schraubenfeder 768, welche sich distalseitig an einem ringförmigen Vorsprung 774 abstützt, welcher von einer Innenwand der Schmiermittelsprayverbindungsleitung 778 vorsteht, in proximaler Richtung gegen einen Ventilsitz 770 gedrückt wird und die Schmiermittelsprayverbindungsleitung 778 in einer Betriebsstellung des Handstücks 700 geschlossen hält. So kann vermieden werden, dass Verunreinigungen vom proximalen Ende 726 durch die Schmiermittelsprayverbindungsleitung 778 hindurch in die Aufnahme 716 gelangen können. Das Ventil 764 ist so dimensioniert, dass die aus dem Druckluftmotor 708 austretende Abluft nicht ausreicht, das Ventil 764 zu öffnen.

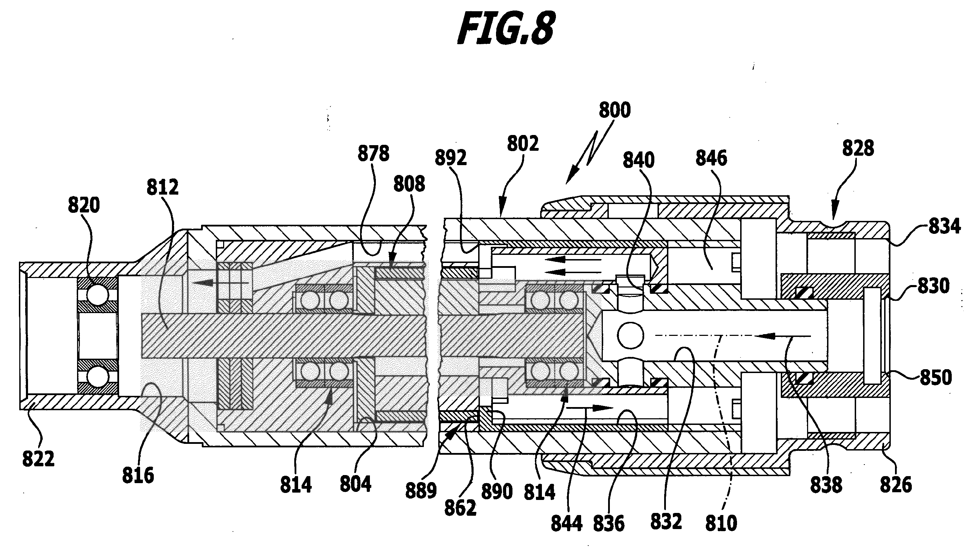

Das chirurgische Handstück 700 kann alternativ auch derart geschmiert werden, dass durch den Drucklufteinlass 730, der dann einen Schmiermittelsprayanschluss 750 bildet, ein Schmiermittelspray eingeleitet wird. Werden bei dieser Vorgehensweise die Druckluftauslässe 734 verschlossen, wird das Ventil 764 durch den so entstehenden Druckaufbau ebenfalls geöffnet und Schmiermittelspray kann durch die Schmiermittelsprayverbindungsleitung 778 in die Aufnahme 716 gelangen. Werden beim Durchsprühen des Handstücks 700 durch den Schmiermittelsprayanschluss 750 hindurch die Druckluftauslässe 734 geöffnet, wird der Druckluftmotor 708 in der üblichen Weise geschmiert. In Figur 8 ist schematisch ein weiteres Ausführungsbeispiel eines chirurgischen Handstücks schematisch dargestellt und insgesamt mit dem Bezugszeichen 800 bezeichnet. Es ist in seinem Aufbau dem Handstück 700 ähnlich.

Koaxial zur Längsachse 810 ist ein Drucklufteinlass 830 ausgebildet, welcher als Schmiermittelsprayanschluss 850 dient. Ein Zuluftkanal 832 ist über einen Querkanal 840 mit einer Zuleitung zum Druckluftmotor 808 verbunden. Die Zuleitung steht wiederum in direkter Fluidverbindung mit einer exzentrisch am Druckluftmotor 808 seitlich vorbeigeführten kanalartigen Schmiermittelsprayverbindungsleitung 878. Eine Einlassöffnung 892 der Schmiermittelsprayverbindungsleitung 878 ist durch ein Schließelement 862 verschließbar, welches um die Längsachse 810 rotierbar am Gehäuse 802 gelagert ist. Es kann von einer Schließstellung, in welcher die Einlassöffnung 892 durch das Schließelement 862 geschlossen ist, in eine Schmierstellung überführt werden, in welcher das Schließelement 862 die Einlassöffnung 892 freigibt, um eine Fluidverbindung zwischen dem Zuluftkanal 832 und der Schmiermittelsprayverbindungsleitung 878 herzustellen. Die Schmierstellung ist schematisch in Figur 8 dargestellt. Ein durch den Schmiermittelsprayanschluss 850 eingeleitetes Schmiermittelspray kann so zum einen durch den Druckluftmotor 808 hindurchströmen, zum anderen aber auch durch die Schmiermittelsprayverbindungsleitung 878 am Druckluftmotor 808 vorbei in die Aufnahme 816, um das Kugellager 820 zu schmieren.

Eine weitere Variante eines chirurgischen Handstücks ist zwar in den Figuren nicht dargestellt, soll jedoch anhand des Handstücks 300 kurz erläutert werden. Wird das Ventil 364 beim Handstück 300 in entgegengesetzter Richtung wirkend in der Rotorwelle 312 ausgebildet, kann das Handstück 300 auch von der unreinen Seite, das heißt direkt durch Einbringen eines Schmiermittelsprays von distal in die Aufnahme 316 hinein geschmiert werden. In diesem Fall kann dann das Ventil öffnen und das Schmiermittelspray in proximaler Richtung in den Zuluftkanal 332 strömen. Wäre beispielsweise in diesem Fall der Drucklufteinlass 330 verschlossen, kann so von distal her auch der mit der Aufnahme 316 in Fluidverbindung stehende Druckluftmotor 308 geschmiert werden.

Fast allen oben beschriebenen Handstücken ist gemein, dass sie eine Schmierung sowohl des Druckluftmotors beziehungsweise dessen Druckluftzelle und des Handstücks im Übrigen mit seinen Lager- und Getriebeteilen mit einem einzigen Sprühstoß ermöglichen. Es ist somit also eine offene, integrierte Bauweise mit einem Druckluftmotor möglich, welche zum einen eine optimale Reinigung des Handstücks ermöglicht, zum anderen aufgrund der vorgeschlagenen Weiterbildungen jedoch auch eine einfache und sichere Schmierung aller relevanten Komponenten der Handstücke. Insbesondere ist eine maschinelle Aufbereitung mit oder ohne angeschlossener Spülung möglich. Hierzu kann optional jeweils der Schmiermittelsprayanschluss des Handstücks mit einer Reinigungsfluidquelle verbunden werden. Dies kann über ein geeignetes Kupplungsstück an einer Reinigungsfluidleitung erfolgen. Das Reinigungsfluid kann dann durch dieselben Hohlräume wie das Schmiermittel ins Innere der Handstücke gelangen und diese so reinigen. Mit anderen Worten bildet, falls gewünscht, jeder Schmiermittelsprayanschluss auch einen Reinigungsfluidanschluss welcher mit dem mindestens einen Lager- oder Getriebeteil und/oder dem Druckluftmotor in Fluidverbindung steht und so eine einfache maschinelle Reinigung der Handstücke ermöglicht.

Eine Schmierung der Handstücke kann dann im Anschluss an die Reinigung und eine gegebenenfalls noch durchgeführte Dampfsterilisation erfolgen, um insbesondere auch korrosionsanfällige Teile der Handstücke zu schmieren und zu schützen und einen möglichst reibungsarmen Betrieb der Handstücke zu gewährleisten. Zur Schmierung wird lediglich ein einziger Sprühkopf benötigt, welcher beispielsweise einstückig mit oder verbunden mit einer Schmiermittelsprayquelle in Form einer Spraydose ausgebildet sein kann.

## Patentansprüche

1. Chirurgisches Handstück (100; 200; 300; 400; 500; 600; 700; 800) zum Antreiben eines chirurgischen Werkzeugs, mit einem Gehäuse (102; 202; 302; 402; 502; 602; 702; 802), in welchem ein Antrieb (106; 206; 306; 406; 506; 606; 706; 806) in Form eines Druckluftmotors (108; 208; 308; 408; 508; 608; 708; 808) und mindestens ein abtriebsseitig mit dem Antrieb (106; 206; 306; 406; 506; 606; 706; 806) zusammenwirkendes Lager- oder Getriebeteil (118; 218; 318; 418; 518; 618; 718; 818) angeordnet sind, welches Handstück (100; 200; 300; 400; 500; 600; 700; 800) mindestens einen Schmiermittelsprayanschluss (148, 150; 248, 250; 348; 448, 450; 548, 550; 648, 650; 748, 750; 848, 850) umfasst zum Koppeln mit einer Schmiermittelsprayquelle (180) und mindestens eine Schmiermittelsprayverbindungsleitung (278; 378; 478; 578; 678; 778; 878) zum Ausbilden einer Fluidverbindung zwischen dem mindestens einen Schmiermittelsprayanschluss (248; 348; 448; 548; 648; 748; 850) und dem mindestens einen Lager- oder Getriebeteil (118; 218; 318; 418; 518; 618; 718; 818), welcher Schmiermittelsprayanschluss (148, 150; 248, 250; 348; 448, 450; 548, 550; 648, 650; 748, 750; 848, 850) mit dem mindestens einen Lager- oder Getriebeteil (118; 218; 318; 418; 518; 618; 718; 818) in Fluidverbindung steht, wobei der mindestens eine Schmiermittelsprayanschluss (150; 248, 250; 348; 448, 450; 548, 550; 648, 650; 748, 750; 848, 850) proximalseitig des Antriebs (206; 306; 406; 506; 606; 706; 806) angeordnet oder ausgebildet ist, **dadurch gekennzeichnet, dass** die mindestens eine Schmiermittelsprayverbindungsleitung (278; 878) seitlich am Antrieb (206; 806) vorbei angeordnet oder ausgebildet ist.

2. Chirurgisches Handstück nach Anspruch 1, **dadurch gekennzeichnet, dass** die mindestens eine Schmiermittelsprayverbindungsleitung (278; 378; 478; 578; 678; 778; 878) in Form einer schlauch- oder rohrförmigen Verbindungsleitung oder in Form eines am Gehäuse (102; 202; 302; 402; 502; 602; 702; 802) angeordneten oder ausgebildeten Verbindungskanals ausgebildet ist.

3. Chirurgisches Handstück nach Anspruch 1 oder 2, **dadurch gekennzeichnet, dass** das Gehäuse (102; 202; 302; 402; 502; 602; 702; 802) mindestens eine Aufnahme (116; 216; 316; 416; 516; 616; 716; 816) für den mindestens einen Lager- oder Getriebeteil (118; 218; 318; 418; 518; 618; 718; 818) umfasst, in welcher Aufnahme (116; 216; 316; 416; 516; 616; 716; 816) der mindestens eine Lager- oder Getriebeteil (118; 218; 318; 418; 518; 618; 718; 818) angeordnet ist, und dass der mindestens eine Schmiermittelsprayanschluss (148; 248; 348; 448; 548; 648; 748, 750; 850) mit der mindestens einen Aufnahme (116; 216; 316; 416; 516; 616; 716; 816) in Fluidverbindung steht.

4. Chirurgisches Handstück nach einem der voranstehenden Ansprüche, **dadurch gekennzeichnet, dass** der mindestens eine Schmiermittelsprayanschluss (150; 248, 250; 348; 448, 450; 548, 550; 648, 650; 748, 750; 848, 850) an einem proximalen Ende des Handstücks (200; 300; 400; 500; 600; 700; 800) angeordnet oder ausgebildet ist.

5. Chirurgisches Handstück nach einem der voranstehenden Ansprüche, **dadurch gekennzeichnet, dass** der mindestens eine Schmiermittelsprayanschluss (148; 348; 648) ein selbstschließendes Einlassventil (164; 364; 664) umfasst.

6. Chirurgisches Handstück nach einem der Ansprüche 2 bis 5, **dadurch gekennzeichnet, dass** der Druckluftmotor (308; 408; 508; 608) eine Rotorwelle (312; 412; 512; 612) umfasst und dass die Rotorwelle die mindestens eine Schmiermittelsprayverbindungsleitung (378; 478; 578; 678) umfasst.

7. Chirurgisches Handstück nach Anspruch 6, **dadurch gekennzeichnet, dass** die Rotorwelle (312; 412; 512; 612) in Form eines hohlen Schafts ausgebildet ist, welcher einen Kanal (388; 488; 588; 688) definiert.

8. Chirurgisches Handstück nach Anspruch 7, **dadurch gekennzeichnet, dass** der Kanal (388; 588; 688) die mindestens eine Schmiermittelsprayverbindungsleitung (378; 578; 678) bildet oder dass die mindestens eine Schmiermittelsprayverbindungsleitung (478) im Kanal (488) angeordnet und relativ zum Gehäuse (402) feststehend ausgebildet ist.

9. Chirurgisches Handstück nach einem der Ansprüche 6 bis 8, **dadurch gekennzeichnet, dass** der mindestens eine Drucklufteinlass (130; 230; 330; 430; 530; 730; 830) koaxial zur Rotorwelle (112; 212; 312; 412; 512; 712; 812) des Druckluftmotors (108; 208; 308; 408; 508; 708; 808) angeordnet oder ausgebildet ist.

10. Chirurgisches Handstück nach einem der Ansprüche 6 bis 9, **dadurch gekennzeichnet, dass** der mindestens eine Druckluftauslass (134; 234; 334; 434; 534; 634; 734; 834) koaxial zur Rotorwelle (112; 212; 312; 412; 512; 612; 712; 812) des Druckluftmotors (108; 208; 308; 408; 508; 708; 808) angeordnet oder ausgebildet ist.

11. Chirurgisches Handstück nach einem der Ansprüche 2 bis 10, **dadurch gekennzeichnet, dass** in oder an der mindestens einen Schmiermittelsprayverbindungsleitung (378; 678; 778; 878) ein in einer Schließstellung druckluftdichtes Schließelement (390; 690; 790; 890) angeordnet oder ausgebildet ist.

12. Chirurgisches Handstück nach Anspruch 11, **dadurch gekennzeichnet, dass** das Schließelement (690; 790; 890) in Form eines selbstschließenden Ventils (664; 764; 864) ausgebildet ist, welches in einer SchmierStellung offenbar ist.

13. Chirurgisches Handstück nach einem der voranstehenden Ansprüche, **gekennzeichnet durch** mindestens einen Schmiermittelsprayanschlussverschluss zum Verschließen des mindestens einen Schmiermittelsprayanschlusses (148, 150; 248, 250; 348; 448, 450; 548, 550; 648, 650; 748, 750; 848, 850).

14. Chirurgisches Handstück nach einem der Ansprüche 10 bis 13, **gekennzeichnet durch** mindestens einen Drucklufteinlass (130; 230, 330; 430; 530; 630; 730; 830) und mindestens einen Druckluftauslass (134; 234; 334; 434; 534; 634; 734; 834), welche mit dem Druckluftmotor (108; 208; 308; 408; 508; 608; 708; 808) in Fluidverbindung stehen, und **durch** ein selbstschließendes Abluftkanalschließventil (764), welches in einer Betriebsstellung geschlossen ist und in einer Schmierstellung geöffnet ist zum Ausbilden einer Fluidverbindung zwischen einem mit dem mindestens einen Druckluftauslass (734) und dem Druckluftmotor (708) verbundenen Abluftkanal und der mindestens einen Schmiermittelsprayverbindungsleitung (778).

15. Chirurgisches Handstück nach einem der voranstehenden Ansprüche, **gekennzeichnet durch** ein mit dem Handstück (100; 200; 300; 400; 500; 600; 700; 800) distalseitig gekoppeltes chirurgisches Werkzeug, insbesondere in Form eines Bohr-, Fräs-, Säge-, Schraub- oder Schneidwerkzeugs.

## Claims

1. Surgical handpiece (100; 200; 300; 400; 500; 600; 700; 800) for driving a surgical tool, with a housing (102; 202; 302; 402; 502; 602; 702; 802), in which there are arranged a drive (106; 206; 306; 406; 506; 606; 706; 806) in the form of a compressed air motor (108; 208; 308; 408; 508; 608; 708; 808) and at least one bearing or gear part (118; 218; 318; 418; 518; 618; 718; 818) cooperating with the drive (106; 206; 306; 406; 506; 606; 706; 806) on the output side, which handpiece (100; 200; 300; 400; 500; 600; 700; 800) comprises at least one lubricant spray connection (148, 150; 248, 250; 348; 448, 450; 548, 550; 648, 650; 748, 750; 848, 850) for coupling to a lubricant spray source (180) and at least one lubricant spray connection conduit (278; 378; 478; 578; 678; 778; 878) for forming a fluidic connection between the at least one lubricant spray connection (248; 348; 448; 548; 648; 748; 850) and the at least one bearing or gear part (118; 218; 318; 418; 518; 618; 718; 818), which lubricant spray connection (148, 150; 248, 250; 348; 448, 450; 548, 550; 648, 650; 748, 750; 848, 850) is in fluidic connection with the at least one bearing or gear part (118; 218; 318; 418; 518; 618; 718; 818), wherein the at least one lubricant spray connection (150; 248, 250; 348; 448, 450; 548, 550; 648, 650; 748, 750; 848, 850) is arranged or configured on the proximal side of the drive (206; 306; 406; 506; 606; 706; 806), **characterized in that** the at least one lubricant spray connection conduit (278; 878) is arranged or configured laterally past the drive (206; 806).

2. Surgical handpiece according to claim 1, **characterized in that** the at least one lubricant spray connection conduit (278; 378; 478; 578; 678; 778; 878) is configured in the form of a flexible tube- or pipe-type connection conduit or in the form of a connection channel arranged or configured on the housing (102; 202; 302; 402; 502; 602; 702; 802).

3. Surgical handpiece according to claim 1 or claim 2, **characterized in that** the housing (102; 202; 302; 402; 502; 602; 702; 802) comprises at least one seating (116; 216; 316; 416; 516; 616; 716; 816) for the at least one bearing or gear part (118; 218; 318; 418; 518; 618; 718; 818), in which seating (116; 216; 316; 416; 516; 616; 716; 816) the at least one bearing or gear part (118; 218; 318; 418; 518; 618; 718; 818) is arranged, and **in that** the at least one lubricant spray connection (148; 248; 348; 448; 548; 648; 748, 750; 850) is in fluidic connection with the at least one seating (116; 216; 316; 416; 516; 616; 716; 816).

4. Surgical handpiece according to any one of the preceding claims, **characterized in that** the at least one lubricant spray connection (150; 248, 250; 348; 448, 450; 548, 550; 648, 650; 748, 750; 848, 850) is arranged or configured on a proximal end of the handpiece (200; 300; 400; 500; 600; 700; 800).

5. Surgical handpiece according to any one of the preceding claims, **characterized in that** the at least one lubricant spray connection (148; 348; 648) comprises a self-closing inlet valve (164; 364; 664).

6. Surgical handpiece according to any one of claims 2 to 5, **characterized in that** the compressed air motor (308; 408; 508; 608) comprises a rotor shaft (312; 412; 512; 612), and **in that** the rotor shaft comprises the at least one lubricant spray connection conduit (378; 478; 578; 678).

7. Surgical handpiece according to claim 6, **characterized in that** the rotor shaft (312; 412; 512; 612) is configured in the form of a hollow shaft, which defines a channel (388; 488; 588; 688).

8. Surgical handpiece according to claim 7, **characterized in that** the channel (388; 588; 688) forms the at least one lubricant spray connection conduit (378; 578; 678) or **in that** the at least one lubricant spray connection conduit (478) is arranged in the channel (488) and is configured to be fixed relative to the housing (402).

9. Surgical handpiece according to any one of claims 6 to 8, **characterized in that** the at least one compressed air inlet (130; 230; 330; 430; 530; 730; 830) is arranged or configured coaxially to the rotor shaft (112; 212; 312; 412; 512; 712; 812) of the compressed air motor (108; 208; 308; 408; 508; 708; 808).

10. Surgical handpiece according to any one of claims 6 to 9, **characterized in that** the at least one compressed air outlet (134; 234; 334; 434; 534; 634; 734; 834) is arranged or configured coaxially to the rotor shaft (112; 212; 312; 412; 512; 612; 712; 812) of the compressed air motor (108; 208; 308; 408; 508; 708; 808).

11. Surgical handpiece according to any one of claims 2 to 10, **characterized in that** a closing element (390; 690; 790; 890) impermeable to compressed air in a closed position is arranged or configured in or on the at least one lubricant spray connection conduit (378; 678; 778; 878).

12. Surgical handpiece according to claim 11, **characterized in that** the closing element (690; 790; 890) is configured in the form of a self-closing valve (664; 764; 864), which is openable in a lubricating position.

13. Surgical handpiece according to any one of the preceding claims, **characterized by** at least one lubricant spray connection closure for closing the at least one lubricant spray connection (148, 150; 248, 250; 348; 448, 450; 548, 550; 648, 650; 748, 750; 848, 850).

14. Surgical handpiece according to any one of claims 10 to 13, **characterized by** at least one compressed air inlet (130; 230; 330; 430; 530; 630; 730; 830) and at least one compressed air outlet (134; 234; 334; 434; 534; 634; 734; 834), which are in fluidic connection with the compressed air motor (108; 208; 308; 408; 508; 608; 708; 808), and by a self-closing exhaust air duct closing valve (764), which is closed in an operating position and is open in a lubricating position for forming a fluidic connection between an exhaust air duct connected to the at least one compressed air outlet (734) and the compressed air motor (708) and the at least one lubricant spray connection conduit (778).

15. Surgical handpiece according to any one of the preceding claims, **characterized by** a surgical tool coupled to the handpiece (100; 200; 300; 400; 500; 600; 700; 800) on the distal side, specifically in the form of a drilling, milling, sawing, screwing or cutting tool.

## Revendications

1. Poignée chirurgicale (100; 200; 300; 400; 500; 600; 700; 800) destinée à l'entraînement d'un outil chirurgical, comprenant un boitier (102; 202; 302; 402; 502; 602; 702; 802) dans lequel sont agencés un entraînement (106; 206; 306; 406; 506; 606; 706; 806) sous la forme d'un moteur à air comprimé (108; 208; 308; 408; 508; 608; 708; 808), et au moins une partie de palier ou de transmission (118; 218; 318; 418; 518; 618; 718; 818) interagissant du côté de sortie d'entraînement avec l'entraînement (106; 206; 306; 406; 506; 606; 706; 806), ladite poignée (100; 200; 300; 400; 500; 600; 700; 800) comportant au moins un raccord de branchement de spray de lubrifiant (148, 150; 248, 250; 348; 448, 450; 548, 550; 648, 650; 748, 750; 848, 850) destiné à être coulé à une source de spray de lubrifiant (180), et au moins une conduite de liaison de spray de lubrifiant (278; 378; 478; 578; 678; 778; 878) pour réaliser une liaison fluidique entre ledit au moins un raccord de branchement de spray de lubrifiant (248; 348; 448; 548; 648; 748; 850) et ladite au moins une partie de palier ou de transmission (118; 218; 318; 418; 518; 618; 718; 818), ledit raccord de branchement de spray de lubrifiant (148, 150; 248, 250; 348; 448, 450; 548, 550; 648, 650; 748, 750; 848, 850) étant en liaison fluidique avec ladite au moins une partie de palier ou de transmission (118; 218; 318; 418; 518; 618; 718; 818), et ledit au moins un raccord de branchement de spray de lubrifiant (150; 248, 250; 348; 448, 450; 548, 550; 648, 650; 748, 750; 848, 850) étant agencé ou formé du côté proximal de l'entraînement (206; 306; 406; 506; 606; 706; 806),
**caractérisée en ce que** ladite au moins une conduite de liaison de spray de lubrifiant (278; 878) est agencée ou formée de manière à longer latéralement l'entraînement (206; 806).

2. Poignée chirurgicale selon la revendication 1, **caractérisée en ce que** ladite au moins une conduite de liaison de spray de lubrifiant (278; 378; 478; 578; 678; 778; 878) est réalisée sous la forme d'une conduite de liaison du type tube ou tuyau, ou sous la forme d'un canal de liaison agencé ou réalisé sur le boitier (102; 202; 302; 402; 502; 602; 702; 802).

3. Poignée chirurgicale selon la revendication 1 ou la revendication 2, **caractérisée en ce que** le boitier (102; 202; 302; 402; 502; 602; 702; 802) comporte au moins un logement d'accueil (116; 216; 316; 416; 516; 616; 716; 816) pour ladite au moins une partie de palier ou de transmission (118; 218; 318; 418; 518; 618; 718; 818), dans ledit logement d'accueil (116; 216; 316; 416; 516; 616; 716; 816) étant agencée ladite au moins une partie de palier ou de transmission (118; 218; 318; 418; 518; 618; 718; 818), et **en ce que** ledit au moins un raccord de branchement de spray de lubrifiant (148; 248; 348; 448; 548; 648; 748, 750; 850) est en liaison fluidique avec ledit au moins un logement d'accueil (116; 216; 316; 416; 516; 616; 716; 816).

4. Poignée chirurgicale selon l'une des revendications précédentes, **caractérisée en ce que** ledit au moins un raccord de branchement de spray de lubrifiant (150; 248, 250; 348; 448, 450; 548, 550; 648, 650; 748, 750; 848, 850) est agencé ou réalisé à une extrémité proximale de la poignée (200; 300; 400; 500; 600; 700; 800).

5. Poignée chirurgicale selon l'une des revendications précédentes, **caractérisée en ce que** ledit au moins un raccord de branchement de spray de lubrifiant (148; 348; 648) comprend une valve d'entrée (164; 364; 664) se fermant automatiquement d'elle-même.

6. Poignée chirurgicale selon l'une des revendications 2 à 5, **caractérisée en ce que** le moteur à air comprimé (308; 408; 508; 608) comprend un arbre de rotor (312; 412; 512; 612), et **en ce que** l'arbre de rotor comprend ladite au moins une conduite de liaison de spray de lubrifiant (378; 478; 578; 678).

7. Poignée chirurgicale selon la revendication 6, **caractérisée en ce que** l'arbre de rotor (312; 412; 512; 612) est réalisé sous la forme d'une tige creuse, qui définit un canal (388; 488; 588; 688).

8. Poignée chirurgicale selon la revendication 7, **caractérisée en ce que** le canal (388; 588; 688) forme ladite au moins une conduite de liaison de spray de lubrifiant (378; 578; 678), ou **en ce que** ladite au moins une conduite de liaison de spray de lubrifiant (478) est agencée dans le canal (488) et est réalisée fixe par rapport au boitier (402).

9. Poignée chirurgicale selon l'une des revendications 6 à 8, **caractérisée en ce que** ladite au moins une entrée d'air comprimé (130; 230; 330; 430; 530; 730; 830) est agencée ou réalisée coaxialement à l'arbre de rotor (112; 212; 312; 412; 512; 712; 812) du moteur à air comprimé (108; 208; 308; 408; 508; 708; 808).

10. Poignée chirurgicale selon l'une des revendications 6 à 9, **caractérisée en ce que** ladite au moins une sortie d'air comprimé (134; 234; 334; 434; 534; 634; 734; 834) est agencée ou réalisée coaxialement à l'arbre de rotor (112; 212; 312; 412; 512; 612; 712; 812) du moteur à air comprimé (108; 208; 308; 408; 508; 708; 808).

11. Poignée chirurgicale selon l'une des revendications 2 à 10, **caractérisée en ce que** dans ou sur ladite au moins une conduite de liaison de spray de lubrifiant (378; 678; 778; 878) est agencé ou réalisé un élément de fermeture (390; 690; 790; 890) étanche à l'air comprimé dans une position fermée.

12. Poignée chirurgicale selon la revendication 11, **caractérisée en ce que** l'élément de fermeture (690; 790; 890) est réalisé sous la forme d'une valve (664; 764; 864) fermant automatiquement d'elle-même, et qui peut être ouverte dans une position de lubrification.

13. Poignée chirurgicale selon l'une des revendications précédentes, **caractérisée par** au moins une fermeture de raccord de branchement de spray de lubrifiant pour assurer la fermeture dudit au moins un raccord de branchement de spray de lubrifiant (148, 150; 248, 250; 348; 448, 450; 548, 550; 648, 650; 748, 750; 848, 850).

14. Poignée chirurgicale selon l'une des revendications 10 à 13, **caractérisée par** au moins une entrée d'air comprimé (130; 230; 330; 430; 530; 630; 730; 830) et au moins une sortie d'air comprimé (134; 234; 334; 434; 534; 634; 734; 834), qui sont en liaison fluidique avec le moteur à air comprimé (108; 208; 308; 408; 508; 608; 708; 808), et par une valve de fermeture de canal d'air d'échappement (764) fermant automatiquement d'elle-même, qui est fermée dans une position de service, et est ouverte dans une position de lubrification pour établir une liaison fluidique entre un canal d'air d'échappement relié à ladite au moins une sortie d'air comprimé (734) et le moteur à air comprimé (708), et ladite au moins une conduite de liaison de spray de lubrifiant (778).

15. Poignée chirurgicale selon l'une des revendications précédentes, **caractérisée par** un outil chirurgical couplé à la poignée (100; 200; 300; 400; 500; 600; 700; 800) du côté distal, et se présentant notamment sous la forme d'un outil de perçage, de fraisage, de sciage, de vissage ou de coupe.
